# FASCICULE DE BREVET EUROPEEN

(11) **EP 1 909 667 B1**
(45) Date de publication et mention de la délivrance du brevet: **13.11.2013**
(21) Numéro de dépôt: 06794515.4
(22) Date de dépôt: 01.08.2006
(51) Int. Cl.: A61B 17/70

(54) **IMPLANT INTERVERTEBRAL A DOUBLE FORME**
DOPPELFÖRMIGES INTERVERTEBRALES IMPLANTAT
DOUBLE-SHAPED INTERVERTEBRAL IMPLANT

(30) Priorité: 04.08.2005 FR 0508321
(43) Date de publication de la demande: 16.04.2008
(73) Titulaire: SCIENT'X, 78284 Guyancourt (FR)
(72) Inventeur: CARLI, Olivier, F-78284 Guyancourt (FR); BERNARD, Pierre, F-33700 Merignac (FR); MAZEL, Christian, F-92100 Boulogne Billancourt (FR); RYAN, David, F-92150 Suresnes (FR)
(74) Mandataire: Thibault, Jean-Marc
(86) Numéro de dépôt international: PCT/FR2006/050774
(87) Numéro de publication internationale: WO 2007/015028

(56) Documents cités:
- WO-A-03/057054
- WO-A-2005/115261
- US-A1- 2004 243 239
- US-A1- 2005 055 031
- US-A1- 2005 131 405

## Description

La présente invention concerne le domaine technique des implants intervertébraux conçus pour stabiliser le rachis selon certaines sollicitations dues aux mouvements du patient telles que la flexion, l'extension, la torsion ou l'inflexion latérale.

L'objet de l'invention vise plus précisément un implant intervertébral comportant une cale destinée à être insérée entre les apophyses épineuses qui prolongent la partie postérieure des vertèbres en vue de limiter leur rapprochement. D'une manière connue, cette cale supplée le disque intervertébral lorsque celui-ci est défaillant de manière à limiter le rapprochement de la partie postérieure des deux vertèbres qui est à même d'induire des douleurs au niveau du rachis.

Dans l'état de la technique, il a été proposé diverses solutions de cales en vue d'éviter le contact entre deux vertèbres. Par exemple, le brevet FR 2 799 640 décrit une cale dans laquelle sont ménagées deux gorges opposées susceptibles de recevoir les deux apophyses épineuses de deux vertèbres. Chaque gorge définie deux ailes qui se trouvent pourvues d'un lien permettant d'entourer une portion de la surface de l'apophyse opposée au fond de la gorge. La pose d'une telle cale nécessite de perforer le ligament interépineux du segment sus-jacent et le ligament interépineux du segment sous-jacent afin de stabiliser la cale.

Il est également connu par la demande de brevet WO 99/21500 une cale interépineuse présentant une forme générale en H et réalisée en deux parties. L'une des parties comporte une aile munie d'un corps d'extension destiné à recevoir l'autre partie présentant une aile. La pose de cette cale nécessite la résection du ligament interépineux permettant une insertion latérale de la cale. Toutefois l'assemblage des deux pièces nécessite un abord bilatéral par rapport aux apophyses épineuses.

Le document US 2005/055031, sur lequel le préambule de la revendication 1 est basé, décrit également un ancillaire pour la mise en place d'un implant intervertébral réalisé en un matériau à mémoire de forme et présentant deux gorges opposées bordées par des ailes de retenue. Cet ancillaire prévoit une pince permettant de rapprocher les deux ailes de retenue pour permettre la mise en place de l'implant. Le retrait de la pince permet aux ailes de revenir à leurs positions initiales. La mise en place de l'implant nécessite l'utilisation d'un ancillaire imposant une voie d'abord importante.

L'objet de l'invention vise à remédier aux inconvénients des solutions techniques antérieures en proposant une cale interépineuse dont la mise en place peut être réalisée par une chirurgie minimale la moins invasive possible.

Cet objectif est atteint par un implant intervertébral selon la revendication 1.

Selon une caractéristique de mise en oeuvre, l'implant intervertébral présente sa forme d'implantation, lorsqu'il est soumis à une chaleur au moins égale à 37°C.

Selon une caractéristique de mise en oeuvre, l'implant intervertébral présente sa forme d'insertion lorsqu'il est soumis à du froid ou à une température ambiante.

Selon un exemple de réalisation, l'implant est dépourvu, dans sa forme d'insertion, des ailes de retenue sur ses deux côtés.

Selon cet exemple, l'implant présente dans sa forme d'insertion, une partie d'insertion formée par les parties formant les gorges et les ailes s'étendant sur les deux côtés des gorges.

Avantageusement, la partie d'insertion présente un profil rectangulaire.

Selon une variante de réalisation, la partie d'insertion présente selon sa hauteur, des conformations du type ressort.

Selon un autre exemple de réalisation, l'implant présente dans sa forme d'insertion, une partie d'insertion formée par les parties formant les gorges et les ailes s'étendant sur l'un des côtés des gorges.

Conformément à un mode de réalisation de l'invention, l'implant est réalisé sous la forme d'un anneau à caractère flexible.

Selon ce mode de réalisation, l'anneau est conformé pour présenter pour chaque gorge une âme prolongée de part et d'autre, par des rebords.

Avantageusement, chaque rebord bordant une gorge est relié au rebord opposé bordant l'autre gorge, par l'intermédiaire d'une branche de raccordement.

Diverses autres caractéristiques ressortent de la description faite ci-dessous en référence aux dessins annexés qui montrent, à titre d'exemples non limitatifs, des formes de réalisation de l'objet de l'invention.
Les **Figures 1** et **1A** sont des vues d'un premier exemple de réalisation d'un implant intervertébral représenté respectivement dans une forme d'implantation et une forme d'insertion.
Les **Figures 2A** à **2C** illustrent trois étapes caractéristiques de la mise en place d'un implant intervertébral illustré aux **Fig. 1** et **1A****.**
Les **Figures 3** et **3A** illustrent un deuxième exemple de réalisation d'un implant intervertébral représenté respectivement dans sa forme d'implantation et sa forme d'insertion.
Les **Figures 4** et **4A** sont des vues schématiques en perspective illustrant un troisième exemple de réalisation d'un implant intervertébral représenté respectivement dans une forme d'implantation et une forme d'insertion.
**Les** **Figures 5A** à **5C** illustrent trois étapes caractéristiques de pose de l'implant intervertébral illustré aux **Fig. 4** et **4A****.**

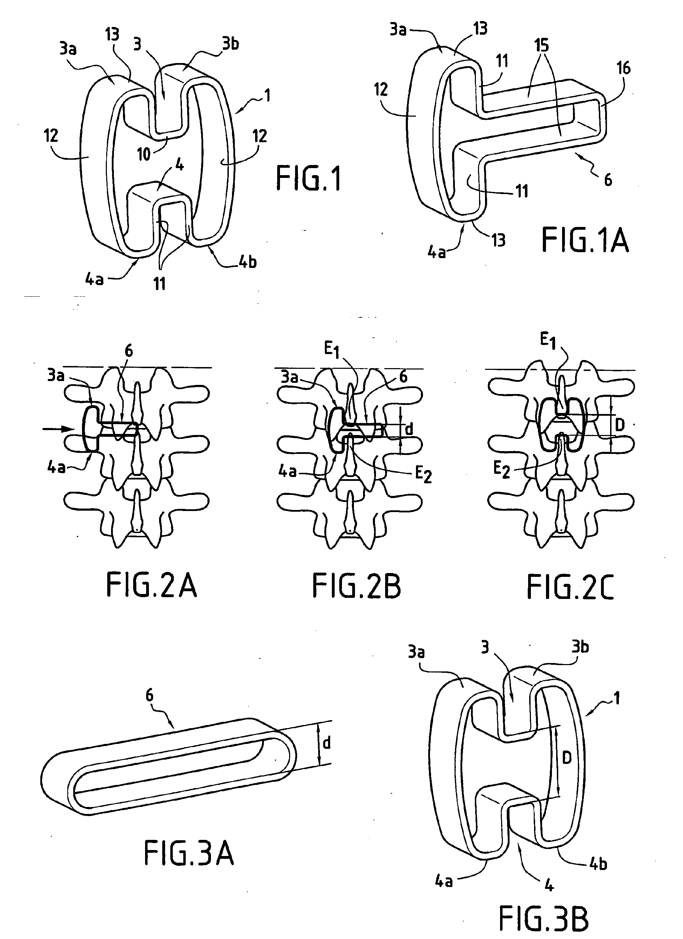

Tel que cela ressort des dessins, l'objet de l'invention concerne un implant intervertébral **1** se présentant sous la forme d'une cale. Conformément à l'invention, l'implant **1** est réalisé en un matériau à mémoire de forme. Par exemple, l'implant **1** est réalisé en un matériau du type Niti plus communément appelé Nitinol. Il doit être compris que l'implant **1** est destiné à présenter au moins deux formes stables à savoir une forme d'insertion telle qu'illustrée aux **Fig. 1A, 3A****,** **4A** et une forme d implantation telle qu'illustrée aux **Fig. 1, 3** et **4****.**

Il doit être considéré que dans sa forme d'implantation, l'implant **1** est soumis à la chaleur du patient c'est-à-dire est soumis à une température au moins de l'ordre de 37°C, voire une température supérieure. Ainsi, la forme d'implantation est définie lors de l'élaboration de l'implant et le matériau est éduqué pour se déformer de façon réversible afin de lui donner une forme d'insertion compatible avec un abord chirurgical unilatéral. L'implant est destiné avant sa pose, afin qu'il prenne sa forme d'insertion, à être soumis à une température inférieure, c'est-à-dire à un refroidissement par rapport à la température conférant à l'implant sa forme d'implantation. Lorsque l'implant **1** est implanté, il se réchauffe au contact des épineuses et retrouve sa forme d'implantation. L'implant **1** conserve cette forme d'implantation tant qu'il reste implanté dans le corps du patient.

Tel que cela apparaît plus précisément dans l'exemple de réalisation illustré aux Fig. **1, 1A, 2A** à **2C,** l'implant **1** présente dans sa forme d'implantation **(****Fig. 1, 2C****),** deux gorges opposées **3** et **4** destinées à recevoir les deux apophyses épineuses **E₁, E₂** de deux vertèbres. Dans la forme d'implantation de l'implant, les gorges **3, 4** sont distantes l'une de l'autre d'une valeur **D** d'écartement des apophyses. Chaque gorge **3, 4** est bordée de part et d'autre, par une aile de retenue respectivement **3a, 3b** et **4a, 4b.**

Tel que cela ressort plus précisément des **Fig. 1A, 2A, 2B****,** l'implant **1** présente dans sa forme d'insertion, une partie d'insertion **6** possédant une hauteur égale à une valeur **d** inférieure à la valeur d'écartement **D** prise dans la forme d'implantation. Il doit être considéré que la partie d'insertion **6** correspond à la partie de l'implant destinée à être engagée latéralement par une unique voie d'abord entre les apophyses épineuses. Cette partie d'insertion **6** possède une hauteur **d** de son extrémité libre ou antérieure jusqu'à une largeur déterminée pour autoriser le déplacement latéral de l'implant jusqu'à ce qu'il occupe sa position d'implantation. Dans l'exemple illustré à la **Fig. 1A****,** cette partie d'insertion **6** correspond aux parties formant les gorges **3, 4** et les ailes **3b, 4b** s'étendant selon le même côté des gorges **3, 4.** Ainsi dans l'exemple illustré, l'implant **1** présente dans sa forme d'insertion, les ailes **3a, 4a** prolongées par la partie d'insertion **6.**

La mise en place d'un implant **1** conforme à l'invention découle directement de la description qui précède. Une incision d'environ 2 à 3 cm est réalisée sur le bord latéral des apophyses épineuses des deux vertèbres concernées. Le cas échéant, cette incision est pratiquée sur le côté où le chirurgien doit pratiquer une décompression sur une racine nerveuse ou sur la moelle épinière. A cet effet, le chirurgien procède au décollement musculaire à partir du bord de l'apophyse épineuse vers les apophyses transverses. Un tel geste permet d'atteindre selon les cas, la hernie discale compressive ou les ostéophytes qui compriment le sac dural. Après avoir procédé à cette décompression médullaire ou radiculaire, la pose de l'implant est réalisée par la même incision en perforant le ligament interépineux tout en préservant le ligament superépineux.

Préalablement à la pose, l'implant **1** est placé à une température lui conférant sa forme d'insertion. L'implant **1** présente sa forme d'insertion lorsqu'il se trouve soumis à la température ambiante, typiquement comprise entre 20 et 25°C, ou à du froid par exemple, par refroidissement par contact avec un gaz inerte tel que de l'azote. Il est procédé ensuite à l'insertion de l'implant qui est introduit par translation dans l'espace interépineux par sa partie d'insertion **6 (****Fig. 2A****)** jusqu'à ce que les ailes **3a, 4a** viennent pratiquement au niveau des apophyses **E₁, E₂ (****Fig. 2B****).** Au contact des épineuses, l'implant **1** est soumis à un réchauffement de sorte qu'il retrouve sa forme initiale dite forme d'implantation. Il est à noter que le réchauffement de l'implant **1** peut être également assuré avec un apport calorifique tel que de l'eau stérile chaude à une température par exemple comprise entre 30 et 50°C.

Lors du retour de l'implant à sa forme définitive d'implantation **(****Fig. 2C****),** la distance entre les épineuses **E₁, E₂** progresse de la valeur **d** à la valeur **D.** Les parties de l'implant formant les gorges **3,4** sont distantes, dans la forme d'implantation, d'une valeur d'écartement **D** supérieure à la valeur de la forme d'insertion, pour assurer l'écartement des apophyses épineuses. Cet écartement des apophyses épineuses a pour effet de soulager les articulaires et la partie postérieure du disque intervertébral. Bien entendu, la valeur de l'écartement **D** est adaptée à la capacité du rachis à accepter cet écartement et varie en fonction de l'étage concerné et de l'amplitude d'écartement souhaité (de 3 à 5 mm par exemple). Il doit être compris que l'implant **1** réalisé en un matériau à mémoire de forme est adapté lorsqu'il est soumis à de la chaleur, à passer d'une forme d'insertion à une forme d'implantation, en assurant un écartement des apophyses épineuses.

Selon une caractéristique avantageuse de réalisation tel qu'illustré aux **Fig. 1** et **1A** l'implant **1** est réalisé sous la forme d'un anneau à caractère flexible. Dans l'exemple illustré, l'anneau est fermé. Bien entendu, il peut être envisagé un anneau ouvert.

Selon cet exemple de réalisation, l'anneau est conformé pour présenter, dans la forme d'implantation, pour chaque gorge **3, 4,** une âme **10** prolongée de part et d'autre sensiblement à l'équerre par des rebords **11.** Les rebords **11** situés d'un même côté des gorges **3, 4** sont reliés ensemble à une branche de raccordement **12** à l'aide de portions de liaison **13.** Chaque portion de liaison **13** possède un contour arrondi orienté en sens contraire de la gorge voisine. La branche de raccordement **12** peut présenter également un profil faiblement courbé. D'une manière générale, un tel anneau ne présente pas d'angle vif.

Dans sa forme d'insertion, l'anneau présente la partie d'insertion **6** délimitée par deux faces planes **15** parallèles entre elles et reliées ensemble par une zone de raccordement **16** de préférence arrondi. A l'opposé de la zone de raccordement **16,** les deux faces planes **15** sont prolongées sensiblement à l'équerre par les rebords **11** qui se trouvent reliés ensemble par une branche de raccordement **12,** à l'aide des portions de liaison **13** de manière à constituer les ailes de retenue **3a, 4a.**

Les Fig. **3** et **3A** illustrent un autre exemple de réalisation d'un implant intervertébral **1** dont la forme d'insertion est différente de la forme d'insertion illustrée à la **Fig. 1A****.** La forme d'implantation de l'implant **1** illustré à la **Fig. 3** est sensiblement la même que la forme d'implantation de l'implant **1** illustré à la **Fig. 1****.** Selon cet exemple de réalisation, l'implant présente dans sa forme d'insertion, une hauteur constante c'est-à-dire un profil rectangulaire. En d'autres termes, l'implant **1** comporte une partie d'insertion **6** de hauteur **d** qui se prolonge sur toute la largeur de l'implant **1.** Ainsi, dans sa forme d'insertion, l'implant **1** ne présente pas les ailes **3a, 4a** ni les ailes **3b, 4b.**

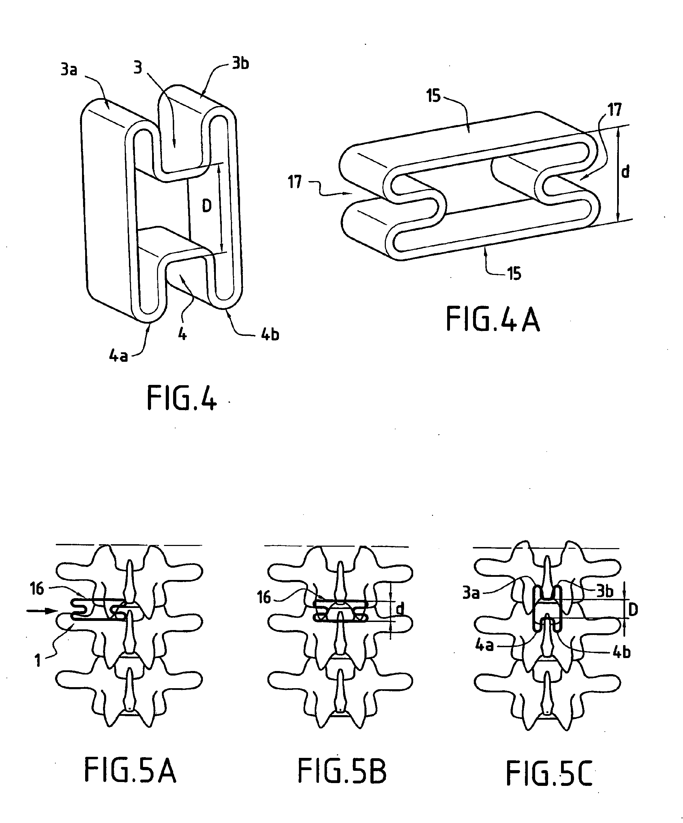

Les **Fig. 4** et **4A** illustrent une autre variante de réalisation dans laquelle l'implant **1** présente une forme d'insertion de forme différente. Dans sa forme d'insertion illustré à la **Fig. 4A****,** l'implant **1** comporte une partie d'insertion **6** présentant une hauteur constante **d** sur toute sa largeur. La partie d'insertion **6** est délimitée par deux faces planes **15.** Dans la position d'insertion **6,** les deux faces planes **15** sont reliées entre elles et à chacune de leurs extrémités, par des conformations **17** telles que des plis en forme d'oméga dans l'exemple illustré **(****Fig. 5A, 5B****).** De telles conformations **17** en forme de ressort contribuent à former les ailes **3a, 3b, 4a, 4b** de l'implant lorsque ce dernier prend sa forme d'implantation illustrée à la **Fig. 4** et à la **Fig. 5C****.**

Il est à noter que quelque soit le mode de réalisation décrit ci-dessus, l'implant **1** présente une capacité d'amortissement obtenu par la flexibilité de l'anneau qui est capable de supporter des variations de charge.

L'invention n'est pas limitée aux exemples décrits et représentés car diverses modifications peuvent y être apportées sans sortir de son cadre.

## Revendications

1. Implant intervertébral comprenant une cale apte à prendre d'une part, une forme d'implantation dans laquelle la cale possède deux gorges opposées **(3, 4)** comportant chacune une âme (10) et bordées par des ailes de retenue **(3a, 3b, 4a, 4b),** les gorges (3, 4) étant destinées à recevoir les deux apophyses épineuses de deux vertèbres et d'autre part, une forme d'insertion dans laquelle les parties de l'implant formant les âmes (10) sont distantes d'une valeur **(d)** et sont dépourvues sur au moins l'un de leurs côtés, des ailes de retenue **(3a, 3b, 4a, 4b), caractérisé en ce que** l'implant **(1)** est réalisé en un matériau à mémoire de forme de manière que l'implant passe à sa forme d'implantation lorsqu'il est soumis à de la chaleur de manière que, dans cette forme d'implantation, les parties de l'implant formant les âmes (10) sont distantes d'une valeur d'écartement **(D)** supérieure à la valeur **(d)** de la forme d'insertion, pour assurer l'écartement des apophyses épineuses.

2. Implant intervertébral selon la revendication 1, **caractérisé en ce qu'**il présente sa forme d'implantation, lorsqu'il est soumis à une chaleur au moins égale à 37°C.

3. Implant intervertébral selon la revendication 1, **caractérisé en ce qu'**il présente sa forme d'insertion lorsqu'il est soumis à du froid ou à une température ambiante.

4. Implant intervertébral selon la revendication 1 ou 3, **caractérisé en ce que** l'implant est dépourvu, dans sa forme d'insertion, des ailes de retenue **(3a, 3b, 4a, 4b)** sur ses deux côtés.

5. Implant intervertébral selon la revendication 4, **caractérisé en ce que** l'implant présente dans sa forme d'insertion, une partie d'insertion **(6)** formée par les parties formant les gorges **(3, 4)** et les ailes **(3a, 3b, 4a, 4b)** s'étendant sur les deux côtés des gorges.

6. Implant intervertébral selon la revendication 5, **caractérisé en ce que** la partie d'insertion **(6)** présente un profil rectangulaire.

7. Implant intervertébral selon la revendication 5, **caractérisé en ce que** la partie d'insertion **(6)** présente selon sa hauteur, des conformations du type ressort **(17).**

8. Implant intervertébral selon la revendication 1 ou 3, **caractérisé en ce que** l'implant présente dans sa forme d'insertion, une partie d'insertion **(6)** formée par les parties formant les gorges **(3, 4)** et les ailes **(3b, 4b)** s'étendant sur l'un des côtés des gorges.

9. Implant intervertébral selon l'une des revendications 1 à 8, **caractérisé en ce que** l'implant est réalisé sous la forme d'un anneau à caractère flexible.

10. Implant intervertébral selon la revendication 9, **caractérisé en ce que** l'anneau est conformé pour présenter pour chaque gorge **(3, 4),** une âme **(10)** prolongée de part et d'autre, par des rebords **(11).**

11. Implant intervertébral selon la revendication 10, **caractérisé en ce que** chaque rebord **(11)** bordant une gorge **(3, 4)** est relié au rebord opposé bordant l'autre gorge, par l'intermédiaire d'une branche de raccordement **(12).**

## Patentansprüche

1. Zwischenwirbelimplantat, umfassend einen Keil, der geeignet ist, einerseits eine Implantationsform anzunehmen, in der der Keil zwei gegenüberliegende Hohlkehlen (3, 4) umfasst, die jeweils einen Kern (10) aufweisen und von Halteflügeln (3a, 3b, 4a, 4b) gesäumt sind, wobei die Hohlkehlen (3, 4) dazu bestimmt sind, die beiden Dornfortsätze von zwei Wirbeln aufzunehmen, sowie andererseits eine Einsetzform anzunehmen, in der die die Kerne (10) bildenden Teile des Implantats um einen Wert (d) beabstandet sind und an wenigstens einer ihrer Seiten nicht die Halteflügel (3a, 3b, 4a, 4b) aufweisen, **dadurch gekennzeichnet, dass** das Implantat (1) aus einem Formgedächtnismaterial ausgebildet ist, so dass das Implantat in seine Implantationsform übergeht, wenn es Wärme ausgesetzt ist, so dass in dieser Implantationsform die die Kerne (10) bildenden Teile des Implantats um einen Abstandswert (D), welcher größer als der Wert (d) der Einsetzform ist, beabstandet sind, um den Abstand der Dornfortsätze sicherzustellen.

2. Zwischenwirbelimplantat nach Anspruch 1, **dadurch gekennzeichnet, dass** es seine Implantationsform aufweist, wenn es einer Wärme von wenigstens gleich 37 °C ausgesetzt ist.

3. Zwischenwirbelimplantat nach Anspruch 1, **dadurch gekennzeichnet, dass** es seine Einsetzform aufweist, wenn es Kälte oder einer Raumtemperatur ausgesetzt ist.

4. Zwischenwirbelimplantat nach Anspruch 1 oder 3, **dadurch gekennzeichnet, dass** das Implantat in seiner Einsetzform an seinen beiden Seiten nicht die Halteflügel (3a, 3b, 4a, 4b) aufweist.

5. Zwischenwirbelimplantat nach Anspruch 4, **dadurch gekennzeichnet, dass** das Implantat in seiner Einsetzform einen Einsetzteil (6) aufweist, der durch die die Hohlkehlen (3, 4) bildenden Teile und die sich auf den beiden Seiten der Hohlkehlen erstreckenden Flügel (3a, 3b, 4a, 4b) gebildet ist.

6. Zwischenwirbelimplantat nach Anspruch 5, **dadurch gekennzeichnet, dass** der Einsetzteil (6) ein rechteckiges Profil aufweist.

7. Zwischenwirbelimplantat nach Anspruch 5, **dadurch gekennzeichnet, dass** der Einsetzteil (6) entlang seiner Höhe federartige Ausbildungen (17) aufweist.

8. Zwischenwirbelimplantat nach Anspruch 1 oder 3, **dadurch gekennzeichnet, dass** das Implantat in seiner Einsetzform einen Einsetzteil (6) aufweist, der durch die die Hohlkehlen (3, 4) bildenden Teile und die sich auf einer der Seiten der Hohlkehlen erstreckenden Flügel (3b, 4b) gebildet ist.

9. Zwischenwirbelimplantat nach einem der Ansprüche 1 bis 8, **dadurch gekennzeichnet, dass** das Implantat in der Form eines Rings flexibler Art ausgebildet ist.

10. Zwischenwirbelimplantat nach Anspruch 9, **dadurch gekennzeichnet, dass** der Ring dazu ausgebildet ist, für jede Hohlkehle (3, 4) einen Kern (10), der auf beiden Seiten durch Ränder (11) fortgesetzt ist, aufzuweisen.

11. Zwischenwirbelimplantat nach Anspruch 10, **dadurch gekennzeichnet, dass** jeder Rand (11), der eine Hohlkehle (3, 4) säumt, mittels eines Verbindungsschenkels (12) mit dem gegenüberliegenden, die andere Hohlkehle säumenden Rand verbunden ist.

## Claims

1. An intervertebral implant that includes a spacer designed firstly to assume an implantation shape in which the spacer has two opposed channels (3, 4) each including a centre part and bordered by retaining wings (3a, 3b, 4a, 4b,), the channels being intended to receive the two spinous processes of two vertebraes, and secondly an insertion shape in which the parts of the implant forming the centre parts (3, 4) are separated by a value (d), and are deprived on at least one of their sides of the retaining wings (3a, 3b, 4a, 4b), **characterised in that** the implant (1) is made from a shape-memory material, so that the implant changes to its implantation shape when it is subjected to heat and so that, in this implantation shape, the parts of the implant forming the centre parts (3, 4) are separated by a value (D) which is greater than the value (d) of the insertion shape, in order to ensure the separation of the spinous processes.

2. An intervertebral implant according to claim 1, **characterised in that** it assumes its implantation shape when it is raised to a temperature of at least 37°C.

3. An intervertebral implant according to claim 1, **characterised in that** it assumes its insertion shape when it is subjected to cold or to ambient temperature.

4. An intervertebral implant according to claims 1 or 3, **characterised in that**, in its insertion shape, the implant is lacking the retaining wings (3a, 3b, 4a, 4b) on its two sides.

5. An intervertebral implant according to claim 4, **characterised in that**, in its insertion shape, the implant has an insertion part (6) formed by the parts forming the channels (3, 4) and the wings (3a, 3b, 4a, 4b) located on the two sides of the channels.

6. An intervertebral implant according to claim 5, **characterised in that** the insertion part (6) has a rectangular profile.

7. An intervertebral implant according to claim 5, **characterised in that**, according to its height, the insertion part (6) has shapes of the spring type (17).

8. An intervertebral implant according to claims 1 or 3, **characterised in that**, in its insertion shape, the implant has an insertion part (6) formed by the parts forming the channels (3, 4) and the wings (3b, 4b) located on one of the sides of the channels.

9. An intervertebral implant according to one of claims 1 to 8, **characterised in that** the implant is created in the form of a flexible ring.

10. An intervertebral implant according to claim 9, **characterised in that**, for each channel, the ring is designed to have (3, 4), a centre part (10) extended on each side by risers (11).

11. An intervertebral implant according to claim 10, **characterised in that** each riser (11) bordering a channel (3, 4) is connected to the opposite riser bordering the other channel by means of a connecting branch (12).
